# EUROPEAN PATENT APPLICATION

(11) **EP 4 657 451 A1**
(43) Date of publication of application: **03.12.2025**
(21) Application number: 24179040.1
(22) Date of filing: 30.05.2024
(51) Int. Cl.: G16H 30/40, G16H 50/70

(54) **MEDICAL IMAGING ALGORITHM INSPECTION**

(71) Applicant: Koninklijke Philips N.V., 5656 AG Eindhoven (NL)
(72) Inventor: WISSEL, Tobias, Eindhoven (NL); NICKISCH, Hannes, Eindhoven (NL); KLINDER, Tobias, 5656AG Eindhoven (NL); GRASS, Michael, Eindhoven (NL)
(74) Representative: Philips Intellectual Property & Standards

(57) **Abstract**

The invention relates to an apparatus comprising at least one processor and at least one memory storing instructions that when executed by the at least one processor cause the apparatus to perform obtaining raw medical image data and calculating a first derivative of an objective function for an algorithm configured to perform an objective. The objective function is dependent on the raw medical image data and a differentiable model for processing the raw medical image data to obtain an image for inputting into the algorithm. The differentiable model is dependent on one or more parameters and the first derivative is calculated with respect to a parameter of the one or more parameters. The apparatus is further caused to perform determining a gradient using the first derivative of the objective function based on the raw medical image data.

## Description

### TECHNOLOGICAL FIELD

Various example embodiments relate to inspecting an algorithm relating to medical image processing.

### BACKGROUND

Medical imaging, such as computed tomography (CT) scanning, is used to help diagnose conditions and monitor patients. The processing solutions for medical imaging are becoming increasingly complex and providing more and more services. For example, predictive machine learning (ML) algorithms may be trained to perform feature recognition within images such that condition diagnosis may be facilitated or automated.

It would be desirable to inspect and monitor the quality of these algorithms.

### BRIEF SUMMARY

The scope of protection sought for various example embodiments of the invention is set out by the independent claims. The example embodiments and features, if any, described in this specification that do not fall under the scope of the independent claims are to be interpreted as examples useful for understanding various embodiments of the invention.

According to various, but not necessarily all, example embodiments of the invention, there is provided an apparatus comprising: at least one processor; and at least one memory storing instructions that when executed by the at least one processor cause the apparatus at least to perform: obtaining raw medical image data; calculating a first derivative of an objective function for an algorithm configured to perform an objective, the objective function being dependent on the raw medical image data and a differentiable model for processing the raw medical image data to obtain an image for inputting into the algorithm, wherein the differentiable model is dependent on one or more parameters and the first derivative is calculated with respect to a parameter of the one or more parameters; and determining a gradient using the first derivative of the objective function based on the raw medical image data.

By calculating a first derivative of the objective function with respect to the parameter of interest using backpropagation, the gradient may be obtained which provides an indication as to the behavior of the algorithm when the parameter is varied. For example, whether a gradient is positive or negative provides an indication as to whether the quality of the result will improve or worsen given a change to the parameter. In some cases, the gradient may provide an indication as to whether the algorithm is more or less likely to fail its objective given a variation in the parameter Accordingly, the invention may provide a simple and quick way to analyze whether an algorithm is capable of coping with a given change to a parameter. The invention may be used to explore the sensitivity of an algorithm with respect to a certain parameter or may be used to notify a user of potential performance degradation due to values for certain parameters being selected for use.

Note that the gradient of the objective function is calculated using the first derivative of the objective function. Since this objective function is dependent on the differentiable model, the objective function will also be differentiable. In this context, a differentiable model means that the model can be expressed as an algorithm(s) which can be differentiated.

In this context, the objective function is for generating an indication of a quality of the output of the algorithm. The objective function may provide a measure of how well, accurately or successfully the algorithm performs its objective.

In some embodiments, the apparatus is further caused to perform: calculating a second derivative of the objective function and determining a curvature using the second derivative based on the raw medical image data.

The gradient provides an indication whether the quality of the result of the algorithm is likely to increase or decrease given an increase or a decrease in the parameter. The curvature provides an indication as to the severity/degree of that change and thus helps provide additional information about the behavior of the algorithm given a variation to the parameter. In other words, the curvature provides an indication whether the increase or decrease in the indication of the quality indicated by the gradient is itself increasing or decreasing. This may allow for a more reliable prognosis when looking into larger changes to the parameter. The curvature is obtained by calculating the second derivative of the objective function.

In some embodiments, the objective function is dependent on reference information, the reference information comprising at least one of the following: a ground truth; and an output of the algorithm when input with the image obtained by processing the raw medical image data using the differentiable model.

The ground truth represents the correct or true result of the objective of the algorithm for a given input image. This may be obtained via manual annotation of the image without the need to pass the image through the algorithm.

The forward pass used to obtain the output of the algorithm may be performed at an operating point which specifies a specific set of values for the one or more parameters. This set of parameters may be well inside the recommended specification range for the algorithm to ensure a valid reference is obtained.

In some embodiments, when the reference information comprises the output of the algorithm, the apparatus is further caused to perform: obtaining the image for inputting into the algorithm by processing the raw medical image data using the differentiable model; and inputting the image into the algorithm to obtain the output.

In some embodiments, objective function comprises a loss function.

Loss is a metric that evaluates the degree of departure of the algorithm output from the reference information or baseline when changing the parameter of interest. The smaller the loss, the more accurate the result of the algorithm. The greater the loss, the further from or more likely to fail its objective. There are many known ways to calculate loss which may be used such as mean squared error (MSE) when performing regression tasks and cross-entropy or Dice score when doing classification or segmentation tasks.

In some embodiments, the algorithm comprises a machine learning algorithm trained to perform the objective.

Note that the weights of the machine learning algorithm are not changed during the process of the invention.

In some embodiments, the objective of the algorithm comprises at least one of the following: a classification task; a detection task; and a segmentation task.

In some embodiments, obtaining the raw medical image data comprises acquiring raw image data using a medical imaging apparatus.

In some embodiments, the medical imaging apparatus comprises at least one of the following: a CT scanner; an MRI scanner; an ultrasound station, an interventional c-arm, cone beam computed tomography (CBCT) apparatus, and x-ray apparatus in general.

In some embodiments, obtaining raw medical image data comprises retrieving the raw medical image data from a data store.

In some embodiments, obtaining raw medical image data comprises estimating the raw medical image data from a pre-existing reconstructed image.

In some embodiments, the raw medical image data comprises a plurality of projections and processing the raw medical image data comprises reconstructing the image using the plurality of projections.

It will be appreciated that there may be additional processing steps to obtain the image in addition to the reconstruction. These steps may be performed before (pre-processing) or after (post-processing) the reconstruction.

In some embodiments, the raw image data comprises computed tomography, CT, projections.

In some embodiments, processing the raw medical image data comprises performing at least one post-processing operation on the reconstructed image.

In some embodiments, the processing comprises manipulating the raw medical image data to model an effect on the raw medical image data.

In some embodiments, the effect comprises at least one of the following: deformation, resolution changes, blurring, motion of a target of the image, detector pixel/module defects, tube arcing, a reduction in a number of samples/projections, an incorrect or incomplete system calibration, a source of noise whether it is known, expected or hypothetical, and image quality degradation.

In some embodiments, the parameter relates to at least one of the following: reconstructing the image using the plurality of projections when the processing comprises reconstructing the image using the plurality of projections; the at least one post-processing operation when the processing comprises at least one post-processing operation; and manipulating the raw image data when the processing comprises manipulating the raw image data.

In other words, the processing may comprise at least one of the following: data manipulation, image formation/reconstruction and post-processing. The parameter of interest may relate to any one of these processing steps if they are present in the processing chain. In some embodiments, a plurality of differentiable models is used to represent the processing chain. For example, one differentiable model may relate to reconstructing the image using the plurality of projections. Another differentiable model may relate to a post-processing operation. Another differentiable model may relate to manipulating the raw medical image data to model an effect on the raw medical image data.

In some embodiments, the parameter relates to at least one of the following: a dose used to acquire the raw image data, detector noise, angular range, a method to combine photo and scatter components of a spectral scan.

In some embodiments, the parameter relates to an image enhancement technique. The image enhancement technique may comprise a filtering step. The filtering step may be for, for example, edge enhancement.

In some embodiments, the parameter is a deterministic parameter which affects the result in a repeatable way.

In some embodiments, the parameter relates to at least one of the following: an angulation range of an imaging device acquiring the raw image data, a reconstruction filter, and a spatial filter.

In some embodiments, the parameter is a random parameter which affects the result in a non-deterministic way.

In some embodiments, the parameter relates to at least one of the following: Gaussian detector noise, and dose-dependent Poisson noise.

In some embodiments, the apparatus is further caused to perform: obtaining an indication of a value of the parameter; and, based on the determined gradient, estimating a quality of an output of the algorithm for a hypothetical image for inputting into the algorithm which would be obtained by processing the raw medical image data using the differentiable model given the value of the parameter.

In this way, the apparatus may predict how the algorithm will behave if the parameter is changed. Estimating the quality of the output for a particular parameter may be a relative quality compared to the operating point at which the reference information is obtained. It will be appreciated that the estimate may further be based on additional derivatives of the objection function, e.g., the curvature.

In some embodiments, the apparatus is further caused to perform: generating for an operator an indication of the estimated quality of the output. In some embodiments, the apparatus is further caused to perform: displaying the indication.

In this way, embodiments may generate an indication to the user whether the algorithm is expected to succeed or fail its objective based on a quality threshold condition for a given parameter.

According to various, but not necessarily all, example embodiments of the invention, there is provided a method comprising: obtaining raw medical image data; calculating a first derivative of an objective function for an algorithm configured to perform an objective, the objective function being dependent on the raw medical image data and a differentiable model for processing the raw medical image data to obtain an image for inputting into the algorithm, wherein the differentiable model is dependent on one or more parameters and the first derivative is calculated with respect to a parameter of the one or more parameters; and determining a gradient using the first derivative of the objective function based on the raw medical image data.

In some embodiments, the method comprises calculating a second derivative of the objective function and determining a curvature using the second derivative based on the raw medical image data.

In some embodiments, the objective function is dependent on reference information, the reference information comprising at least one of the following: a ground truth; and an output of the algorithm when input with the image obtained by processing the raw medical image data using the differentiable model.

In some embodiments, when the reference information comprises the output of the algorithm, the method comprises: obtaining the image for inputting into the algorithm by processing the raw medical image data using the differentiable model; and inputting the image into the algorithm to obtain the output.

In some embodiments, the objective function comprises a loss function.

In some embodiments, the algorithm comprises a machine learning algorithm trained to perform the objective.

In some embodiments, the objective of the algorithm comprises at least one of the following: a classification task; a detection task; and a segmentation task.

In some embodiments, obtaining the raw medical image data comprises acquiring raw image data using a medical imaging apparatus.

In some embodiments, the raw medical image data comprises a plurality of projections and processing the raw medical image data comprises reconstructing the image using the plurality of projections.

In some embodiments, processing the raw medical image data comprises performing at least one post-processing operation on the reconstructed image.

In some embodiments, the processing comprises manipulating the raw medical image data to model an effect on the raw medical image data.

In some embodiments, the parameter relates to at least one of the following: reconstructing the image using the plurality of projections when the processing comprises reconstructing the image using the plurality of projections; the at least one post-processing operation when the processing comprises at least one post-processing operation; and manipulating the raw image data when the processing comprises manipulating the raw image data.

In some embodiments, the method comprises: obtaining an indication of a value of the parameter; and, based on the determined gradient, estimating a quality of an output of the algorithm for a hypothetical image for inputting into the algorithm which would be obtained by processing the raw medical image data using the differentiable model given the value of the parameter.

In some embodiments, the method further comprises the steps described in the various embodiments of the apparatus.

According to various, but not necessarily all, example embodiments of the invention, there is provided a computer program comprising instructions which, when executed by an apparatus, cause the apparatus to perform at least the following: obtaining raw medical image data; calculating a first derivative of an objective function for an algorithm configured to perform an objective, the objective function being dependent on the raw medical image data and a differentiable model for processing the raw medical image data to obtain an image for inputting into the algorithm, wherein the differentiable model is dependent on one or more parameters and the first derivative is calculated with respect to a parameter of the one or more parameters; and determining a gradient using the first derivative of the objective function based on the raw medical image data.

The instructions may be for performing the optional features set out in relation to the method mentioned above.

Furthermore, an embodiment of the invention may also relate to a computer-readable (storage) medium that comprises instructions which, when executed by a computer, cause the computer to carry out (any of the steps of) any of the methods described above.

Further particular and preferred aspects are set out in the accompanying independent and dependent claims. Features of the dependent claims may be combined with features of the independent claims as appropriate, and in combinations other than those explicitly set out in the claims.

Where an apparatus feature is described as being operable to provide a function, it will be appreciated that this includes an apparatus feature which provides that function or which is adapted or configured to provide that function.

### BRIEF DESCRIPTION

Some example embodiments will now be described with reference to the accompanying drawings in which:
Fig. 1 illustrates variations in the appearance of an image given variations of certain parameters;
Fig. 2 illustrates a system according to an embodiment; and
Fig. 3 illustrates steps in a method according to an embodiment.

### DETAILED DESCRIPTION

Before discussing the example embodiments in any more detail, first an overview will be provided.

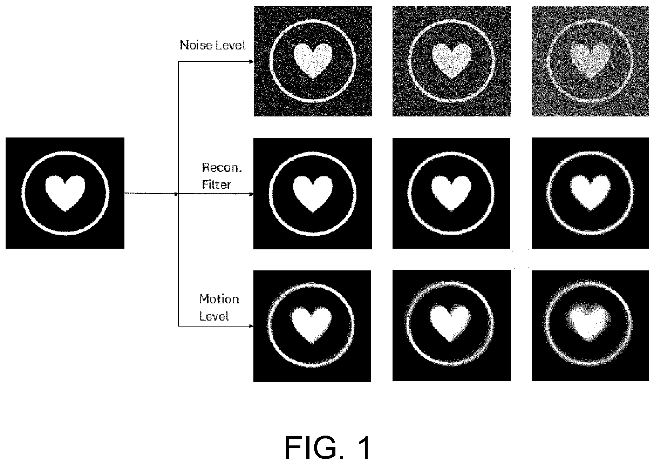

Medical image acquisition, formation and post-processing are subject to a growing set of parameters which configure their behavior and the appearance of the final image. For example, during a computed tomography (CT) scan, projections (raw image data) are acquired which are subsequently used to reconstruct an image. This image may be subject to additional post-processing procedures. CT reconstructions may differ in appearance and image quality depending on, for instance, dose configurations (where lower radiation doses entail an increase in Poisson noise in the projections), modifying the angular range for reconstructions, specific combinations of spectral components, or parameters of image enhancement algorithms (e.g., noise reduction), etc. Fig. 1 illustrates the different appearances of an image depending on various configurations. In this case, variations in noise level, the reconstruction filter used and a motion level are shown.

A model, based on a predictive algorithm and trained on a given dataset, risks relying too much on a certain parameter subspace of the image formation and processing chain and is therefore a "narrow expert" which is prone to certain failure modes caused by departing from this subspace. In other words, the model may be unable to perform its objective when a parameter on which the image formation is dependent is changed. As a result, the model/algorithm may fail to generalize to broader applicability. Note that the model may be trained to perform an objective when given a medical image as an input. For example, the model/algorithm may be configured to diagnose a condition or to determine the presence of a feature within the image (e.g., pathology classification, organ segmentation etc.).

Parameters of the image formation and processing chain may vary due to changes in the acquisition protocol (e.g., dose reduction in CT, contrast injections, timings, etc.) or due to changing characteristics of the imaged patient (e.g., motion of contrasted vessels). To identify and compensate for relevant failure modes, the predictive algorithm needs to be inspected by observing and assessing its behavior given various examples of the modes to be tested. This may happen during algorithm development or post market surveillance and is traditionally performed using either real data with non-disentangled characteristics (i.e., a plurality of real results are obtained having different parameters to see whether the algorithm can handle the changes) or by compute-intense simulation of point estimates (i.e., the image processing is simulated given a variety of parameter values and multiple results are obtained using the algorithm for each parameter value to see whether the algorithm can handle the changes).

Such assessments require the availability of suitable data to (train and) test on and thus usually require significant additional effort. For real data, this can be challenging as examples for certain failure modes are rarely acquired and may be entangled with changes of other characteristics/parameters at the same time. In some cases, real results cannot easily be obtained because it would for instance require exposing the patient to even more dose. Alternatively, after the acquisition of raw data, if the reconstruction and/or post-processing of the final image is simulated under different parameterizations, this may be very computational, memory and time intense as examples for each parameterization need to be explicitly generated in order to pass them through the model for evaluation. This challenge is even bigger when parameters affect a random variable (such as dose which affects image noise), for which ideally more than one sample of the same parameterization needs to be generated for a thorough assessment of the model behavior.

There is a need for algorithm analysis which can provide information about use cases and parameterizations for which the developed algorithm can be used safely and for which it cannot. Moreover, not all potential failure modes can be explored exhaustively and may therefore require post-market surveillance. For instance, the model behavior may not only depend on the chosen dose as such, but also on the contents of the current image. The user may not generally be informed of the expected performance change of the predictive algorithm based on the parameters of the image formation alone. So, there is a potential need to perform this assessment while the software is in the field.

Embodiments propose modelling an image processing chain, performed on raw medical image data, for obtaining an image for inputting into an algorithm configured to perform an objective. The image processing chain is modelled using a differentiable model which is dependent on one or more parameters. As a result, the objective function (e.g., a loss function) used to analyze the performance of the algorithm will also be differentiable because it will be dependent on the differentiable model. The objective function is then differentiated with respect to a parameter of interest to obtain the first derivative of the objective function. From this, a gradient may be determined based on the raw medical image data and baseline parameters. This gradient provides an indication as to whether the quality of the result will increase or decrease given an increase or a decrease in the parameter of interest without the need for simulating any changes to the parameter (i.e., it is not necessary to input different parameter values into the model of the processing chain, pass each variation through the algorithm and analyze each result using the objective function). In some embodiments, where possible, a second derivative of the objective function with respect to the parameter of interest is calculated and from this a curvature may be determined based on the raw medical image data and baseline parameter values. The curvature provides an indication of how quickly the gradient of the objective function will change given a variation in the parameter of interest.

In some embodiments, the objective function may be dependent on reference information. The reference information may be a ground truth that is obtained through manual input (i.e., the image is not input into the algorithm, instead the true result of the objective is manually given by a user). In other embodiments, the reference information may be taken to be the result of the algorithm when input with the image generated by the processing chain based on the raw medical image data and the baseline parameters. In other embodiments, the loss function may be some form of qualitative assessment in which case no reference information may be needed.

By determining the gradient and in some cases further derivatives, indications as to the behavior of the algorithm given changes to a parameter of interest may be obtained. In this way, embodiments may provide a quick and effective inspection of an algorithm in relation to a disentangled parameter of interest. It will be appreciated that embodiments may not provide as much detail as to how the algorithm behaves due to changes in the parameter of interest because the quality (e.g., the loss) of the result of the algorithm is not explicitly determined for every parameter value. However, by avoiding the impracticality of performing multiple real data collections using different parameter values and avoiding passing multiple simulated images generated using different parameter values through the algorithm, a simplified inspection of the algorithm may be performed which may be faster and require fewer resources.

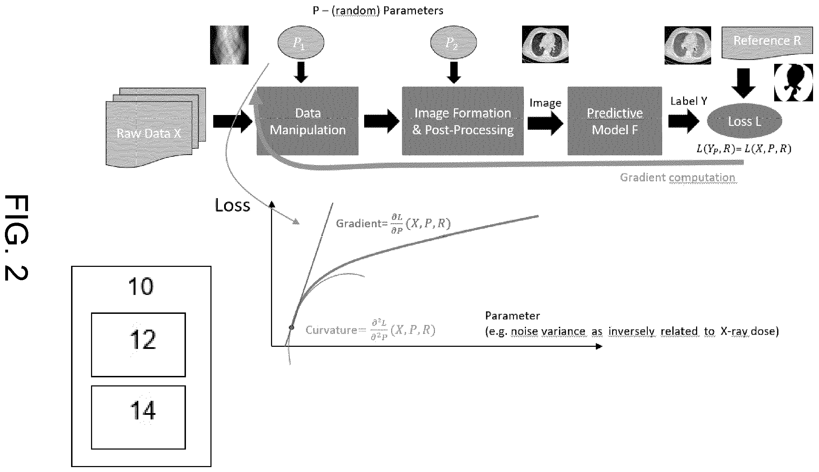

Fig. 2 discloses a system according to an embodiment. The system comprises a first module for obtaining raw medical image data, X. The raw medical image data X may be obtained by acquiring the raw data using a medical imaging apparatus such as a CT scanner or an MRI scanner. Alternatively, the raw medical image data X may be retrieved from a data store or estimated from a pre-existing reconstructed image.

Raw data refers to the output directly after acquisition. For a CT image, this would be the projection data with the accompanying meta information (e.g., angulation, cardiac phase etc.). Alternatively, estimation of original raw data might be generated by forward projecting an existing reconstructed image.

Next, the raw image data X is passed through a processing chain which is dependent on one or more parameters. In this embodiment, the processing chain comprises a data manipulation module, an image formation (e.g., reconstruction) module combined with a post-processing module.

Data manipulation is performed before the raw image data enters the image formation (e.g., CT reconstruction) and post-processing stage. Data manipulation may have the form of simulating certain variations/effects via a forward model, such as motion warping, which would normally originate from the imaged object. In other words, the data manipulation models any effect that would happen to the raw data before reconstruction into the image space. The data manipulation is dependent on one or more parameters, P1. In some embodiments, data manipulation models an effect on the raw medical image data. The effect may comprise at least one of the following: deformation, resolution changes, blurring, motion of a target of the image (e.g. due to target breathing), detector pixel/module defects, tube arcing, a reduction in a number of samples/projections, an incorrect or incomplete system calibration, a source of noise whether it is known, expected or hypothetical (electronics imperfection, detector noise, etc.), and image quality degradation (artifacts, contrast level, etc.).

Data manipulation may also involve an intermediate reconstruction (i.e. transformation into another representation) - for example, the X-ray projections may be reconstructed to obtain a 3D representation (intermediate CT image), which is then warped by N different motion vector fields (MVFs) simulating different motion states from which projections are generated in such a way that not all projections come from the same MVF and hence motion artifacts are generated in the upcoming reconstruction. The scale of the motion vectors can serve as a deterministic parameter (or random if the distribution of scales can be defined).

Next, in the image formation stage, the image for inputting into the algorithm is formed. For example, the raw medical image data may comprise a plurality of (e.g., CT) projections and the image formation comprises reconstructing the plurality of projections to obtain the image. The image may undergo some post-processing. The image formation and any post-processing stages may be dependent on one or more parameters, P2.

The parameters on which the reconstruction is dependent may include dose (parametrizing the amount of Poisson noise), detector noise (parametrizing the amount of Gaussian noise on the image), the angular range (parametrizing e.g., the (down)weighting of projections in certain angle intervals), or the way photo and scatter components of a spectral scan are combined into the final image.

The post-processing may include for instance filtering steps (e.g., for edge enhancements) or other image enhancement techniques.

It will be appreciated that the processing chain may comprise more or fewer steps than described dependent on the nature of the raw medical image data and the required format of the image for inputting into the algorithm. For example, in some embodiments, the data manipulation stage may not be present. In some embodiments, the image formation stage may not be present. In some embodiments, the post-processing stage may not be present.

The one or more parameters on which the processing chain may be dependent on may be deterministic parameters that control the final image output in a deterministic way when repeated (e.g., angulation range, reconstruction filter or post-processing parameters like spatial filters). Alternatively, they may be random parameters that control output generation in a non-deterministic way such as parameters of a probability distribution from which is sampled (e.g., Gaussian detector noise or dose-dependent Poisson noise).

A random distribution affecting the pipeline may be expressed using the re-parameterization trick [3] such that the random sampling is isolated into a unit distribution and the parameter is analytically affecting it, so that gradients can be computed with respect to distribution parameters. For normal distributions with parameters µ and σ , this would result in x - N(µ, σ) = x ~ N(0,1)·σ + µ. For discrete distributions such as the Poisson distribution, the generalized Gumbel-Softmax approximation can be used [4]. For the operating point at which the degradation curve parameters are approximated, random distributions can be reduced to their expectation. Examples for these parameters are given in the respective modules below.

The resulting image output of the processing chain may be passed to the predictive model, which is configured to perform the task/objective it was trained for, in this case to produce a label. The predictive model may be a separately trained model fulfilling a particular high-level task such as classification, detection, or segmentation for which the failure modes (on given input data) shall be assessed. Note that the network and its parameters/weights are considered fixed during the entire process and are solely a processing step through which the gradients are computed.

A measure of the quality of the result/output of the algorithm may be determined using one or more objective functions. In this instance, the loss is calculated using a loss function that is dependent on the raw image data, the processing chain (and the parameters on which this depends) and reference information, R. Note that loss is a metric that evaluates the departure of the pipeline output from the baseline when changing the parameter of interest. The reference information R may be an available ground truth information. Alternatively, the reference information R may be the baseline output from the pipeline when the input is the current operating point (i.e., the input image at hand), e.g., when using a higher dose during CT imaging which implies a lower noise level. This could be, for instance, a set of parameters that is well within the recommended parameter range from the vendor. However, it will be appreciated that these parameters may not necessarily be the same in all scenarios and that one may want to observe as a user whether the output of the algorithm is likely to be worse when the parameters used are 'off-label'.

To inspect the algorithm, embodiments simulate the processing chain using one or more differentiable models which are dependent on one or more parameters, e.g., P1 and P2. For example, the differentiable reconstruction process into image space and exemplary subsequent post-processing may be expressed/approximated by a differentiable algorithm as disclosed in [1] and [2]. Given that the loss function depends on the output of the algorithm which in turn depends on the processing chain, the loss function will also be differentiable.

In a backward pass, the gradient of the loss function (which may, for example, compare the output label and a reference) is computed with respect to a certain parameter of interest to approximate the slope of the loss degradation curve as shown in the graph. The curvature can be approximated by the second derivative of the loss function with respect to the parameter of interest. If possible, further derivatives may be calculated to obtain further information indicative of the behavior of the loss function. These derivatives describe the behavior of the loss curve without computing it exactly (i.e., embodiments do not need to generate costly samples across the parameter space as discussed above).

In some embodiments, the system estimates a quality of an output of the algorithm based on the gradient (and further derivatives if calculated) for an image input into the algorithm which has been obtained by processing the raw medical image data using the differentiable model where the parameter has been changed.

In some embodiments, the system generates for an operator an indication of the estimated quality of the potential output for a given value of a parameter. In some embodiments, the system displays the indication. Thus, embodiments may generate an indication to the user whether the algorithm is expected to succeed or fail its objective based on a quality threshold condition for a given parameter. Accordingly, a user may be able to enquire the system as to whether the algorithm will be able to perform its objective given a certain set of parameters.

In summary, embodiments output indicators that describe the performance curve for a pre-selected parameter to the user. This may be performed on the fly when (or before) the acquisition is carried out in the hospital or during algorithm testing at the manufacturer site. In the latter case, outputs from the invention may be used as information provided to regulatory bodies. In some embodiments, the disclosed methods are applied during product and algorithm development to understand weaknesses of a current predictive model and its behavior under certain failure modes. In some embodiments, this information is used to define boundary conditions for the intended use or even as a documentation for regulatory bodies.

Alternatively, the invention can be applied for regular submission or during deployment, e.g., when, for a given system parameterization, it needs to be understood (on-the-fly) whether a certain change in parameters (e.g., lowering the dose) would impact the performance of the predictive algorithm (for this patient) or whether it can be safely applied. It can also play a role during clinical decision making, e.g., by helping the clinician to understand whether medication for sedation or heart rhythm control can be relaxed as the predictive model output will hardly be affected by an additional amount of motion in the imaged field of view (FoV).

In an example embodiment, the system is implemented by an apparatus 10 comprising at least one processor 12 and at least one memory 14. The memory 14 stores instructions that when executed by the at least one processor 12 cause the apparatus 10 at least to perform the actions according to the embodiments discussed above. It will be appreciated that embodiments of the invention may be carried out by any other suitable circuitry, instructions and means.

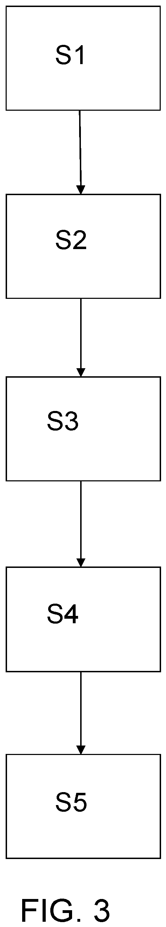

Fig. 3 illustrates the steps in a method according to an embodiment. The method may be performed by the apparatus 10 described above.

Step 1 comprises obtaining raw medical image data.

Step 2 is optional and comprises obtaining an image for inputting into an algorithm configured to perform an objective by processing the raw medical image data using a differentiable model. The differentiable model is dependent on one or more parameters.

Step 2 is only performed when an objective function for the algorithm is dependent on reference information and when the reference information comprises an output of the algorithm when input with the image obtained by processing the raw medical image data using the differentiable model.

Step 3 is optional and comprises inputting the image into the algorithm to obtain the output. Step 3 is only performed when step 2 is performed.

Step 4 comprises calculating a first derivative of the objective function for the algorithm configured to perform the objective. The objective function is dependent on the raw medical image data and the differentiable model for processing the raw medical image data to obtain the image for inputting into the algorithm. The first derivative is calculated with respect to a parameter of the one or more parameters.

Step 4 optionally comprises calculating a second derivative of the objective function with respect to the parameter of the one or more parameters and determining a curvature using the second derivative based on the raw medical image data.

It will be appreciated that step 4 may be performed before or at the same time as steps 2 and 3.

Step 5 comprises determining a gradient using the first derivative of the objective function based on the raw medical image data. When the objective function for the algorithm is dependent on the reference information, the determination of the gradient is also based on the reference information.

In other words, the invention relates to medical image processing solutions which are providing more and more complex services to the customer. This trend challenges traditional image processing techniques and entails a shift towards fully or partially data-driven approaches. These "learn" by optimizing objectives on samples of data instead of explicitly defining them.

These approaches bear the risk of relying too much on a parameter subspace of the image formation chain and being prone to certain failure modes caused by departing from this path. This may either happen due to changes in the acquisition protocol (e.g., dose reduction in CT scanning or number of sample reduction in MR scanning) or due to changing characteristics of the imaged patient (e.g., motion of contrasted vessels). To compensate for relevant failure modes, the predictive algorithm (e.g., pathology classification, organ segmentation etc.) needs to be inspected by observing and assessing its behavior on various examples related to the modes to be tested. This may happen during algorithm development or post market surveillance by either using real data with non-disentangled characteristics or by compute-intense simulation of point estimates.

The invention proposes to leverage differentiable implementations of the corresponding image formation pipeline (e.g., CT image reconstruction from projection data) and/or post-processing chain to compute gradients of the objective/loss function used to optimize the predictive algorithm with respect to parameters of the image formation/post-processing chain. These parameters can be a deterministically related to the prediction output or can be random variables such as certain types of noise. The distributions of the latter are reparametrized by the invention to be able to compute the gradients with respect to the distributional parameters. The parameters of the predictive model remain fixed.

The invention solves the challenges described above by directly estimating the impact of parameter changes in the image formation / post-processing chain given (1) the trained Predictive Model and its optimization objective, (2) raw input data for the relevant modality, (3) a differentiable model of the image formation / post processing, and (4) differentiable simulations of relevant factors influencing the image appearance (e.g. a noise model or a motion model).

The invention makes use of these differentiable set of models to compute gradients of the objective function at the current raw data sample with respect to either deterministic or random input parameters to the image formation or post-processing chain. These gradients can describe the functional relationship of the loss with respect to the respective parameter, without explicitly computing or acquiring the corresponding images and computing the objective value after passing each of them through the network. The curve of potential performance degradation is therefore not explicitly computed, but properties of it are estimated. Fig. 2 illustrates the components and the described process.

The invention can be used to (1) explore based on a given set of data the sensitivity of the predictive algorithm with respect to a certain failure mode, or (2) to notify the user of expected performance degradation the predictive algorithm will exhibit when changing certain parameters of the acquisition protocol for the current acquisition (e.g., lowering the dose).

A person of skill in the art would readily recognize that steps of various above-described methods can be performed by programmed computers. Herein, some embodiments are also intended to cover program storage devices, e.g., digital data storage media, which are machine or computer readable and encode machine-executable or computer-executable programs of instructions, wherein said instructions perform some or all of the steps of said above-described methods. The program storage devices may be, e.g., digital memories, magnetic storage media such as a magnetic disks and magnetic tapes, hard drives, or optically readable digital data storage media. The embodiments are also intended to cover computers programmed to perform said steps of the above-described methods. The tern non-transitory as used herein, is a limitation of the medium itself (i.e., tangible, not a signal) as opposed to a limitation on data storage persistency (e.g. RAM vs ROM).

As used in this application, the term "circuitry" may refer to one or more or all of the following:
(a) hardware-only circuit implementations (such as implementations in only analog and/or digital circuitry) and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions) and
(c) hardware circuit(s) and or processor(s), such as a microprocessor(s) or a portion of a microprocessor(s), that requires software (e.g., firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor (or multiple processors) or portion of a hardware circuit or processor and its (or their) accompanying software and/or firmware. The term circuitry also covers, for example and if applicable to the particular claim element, a computing device.

As used herein, "at least one of the following: <a list of two or more elements>" and "at least one of <a list of two or more elements>" and similar wording, where the list of two or more elements are joined by "and" or "or", mean at least any one of the elements, or at least any two or more of the elements, or at least all the elements.

The ordering of method steps set out above may not be critical or fixed and the exact ordering of the steps may be varied as appropriate.

Although example embodiments of the present invention have been described in the preceding paragraphs with reference to various examples, it should be appreciated that modifications to the examples given can be made without departing from the scope of the invention as claimed.

Features described in the preceding description may be used in combinations other than the combinations explicitly described.

Although functions have been described with reference to certain features, those functions may be performable by other features whether described or not.

Although features have been described with reference to certain embodiments, those features may also be present in other embodiments whether described or not.

Whilst endeavoring in the foregoing specification to draw attention to those features of the invention believed to be of particular importance it should be understood that the Applicant claims protection in respect of any patentable feature or combination of features hereinbefore referred to and/or shown in the drawings whether or not particular emphasis has been placed thereon.

### REFERENCES

[1] Ronchetti, M.. (2020). TorchRadon: Fast Differentiable Routines for Computed Tomography.
[2] Wang, G., Luo, T., Nielsen, J.F., Noll, D., & Fessler, J. (2022). B-spline Parameterized Joint Optimization of Reconstruction and K-space Trajectories (BJORK) for Accelerated 2D MRI. IEEE Transactions on Medical Imaging, 1-1.
[3] Kingma, D., & Welling, M. (2013). Auto-Encoding Variational Bayes. arXiv e-prints, arXiv: 1312.6114.
[4] Joo, Weonyoung et al. "Generalized Gumbel-Softmax Gradient Estimator for Various Discrete Random Variables." ArXiv abs/2003.01847 (2020)

## Claims

1. An apparatus comprising:
at least one processor; and
at least one memory storing instructions that when executed by the at least one processor cause the apparatus at least to perform:
obtaining raw medical image data;
calculating a first derivative of an objective function for an algorithm configured to perform an objective, the objective function being dependent on the raw medical image data and a differentiable model for processing the raw medical image data to obtain an image for inputting into the algorithm,
wherein the differentiable model is dependent on one or more parameters and the first derivative is calculated with respect to a parameter of the one or more parameters; and
determining a gradient using the first derivative of the objective function based on the raw medical image data.

2. An apparatus according to claim 1, wherein the apparatus is further caused to perform:
calculating a second derivative of the objective function and determining a curvature using the second derivative based on the raw medical image data.

3. An apparatus according to claim 1 or claim 2, wherein the objective function is dependent on reference information, the reference information comprising at least one of the following:
a ground truth; and
an output of the algorithm when input with the image obtained by processing the raw medical image data using the differentiable model.

4. An apparatus according to claim 3, wherein, when the reference information comprises the output of the algorithm, the apparatus is further caused to perform:
obtaining the image for inputting into the algorithm by processing the raw medical image data using the differentiable model; and
inputting the image into the algorithm to obtain the output.

5. An apparatus according to any preceding claim, wherein the objective function comprises a loss function.

6. An apparatus according to any preceding claim, wherein the algorithm comprises a machine learning algorithm trained to perform the objective.

7. An apparatus according to any preceding claim, wherein the objective of the algorithm comprises at least one of the following:
a classification task;
a detection task; and
a segmentation task.

8. An apparatus according to any preceding claim, wherein obtaining the raw medical image data comprises acquiring raw image data using a medical imaging apparatus.

9. An apparatus according to any preceding claim, wherein the raw medical image data comprises a plurality of projections and processing the raw medical image data comprises reconstructing the image using the plurality of projections.

10. An apparatus according to claim 9, wherein processing the raw medical image data comprises performing at least one post-processing operation on the reconstructed image.

11. An apparatus according to any preceding claim, wherein the processing comprises manipulating the raw medical image data to model an effect on the raw medical image data.

12. An apparatus according to any one of claims 9 to 11, wherein the parameter relates to at least one of the following:
reconstructing the image using the plurality of projections when the processing comprises reconstructing the image using the plurality of projections;
the at least one post-processing operation when the processing comprises at least one post-processing operation; and
manipulating the raw image data when the processing comprises manipulating the raw image data.

13. An apparatus according to any preceding claim, wherein the apparatus is further caused to perform: obtaining an indication of a value of the parameter; and, based on the determined gradient, estimating a quality of an output of the algorithm for a hypothetical image for inputting into the algorithm which would be obtained by processing the raw medical image data using the differentiable model given the value of the parameter.

14. A method comprising the steps of:
obtaining raw medical image data;
calculating a first derivative of an objective function for an algorithm configured to perform an objective, the objective function being dependent on the raw medical image data and a differentiable model for processing the raw medical image data to obtain an image for inputting into the algorithm,
wherein the differentiable model is dependent on one or more parameters and the first derivative is calculated with respect to a parameter of the one or more parameters; and
determining a gradient using the first derivative of the objective function based on the raw medical image data.

15. A computer program comprising instructions which, when the program is executed by a computer, cause the computer to carry out at least the steps of the method according to claim 14, in particular the steps of:
obtaining raw medical image data;
calculating a first derivative of an objective function for an algorithm configured to perform an objective, the objective function being dependent on the raw medical image data and a differentiable model for processing the raw medical image data to obtain an image for inputting into the algorithm,
wherein the differentiable model is dependent on one or more parameters and the first derivative is calculated with respect to a parameter of the one or more parameters; and
determining a gradient using the first derivative of the objective function based on the raw medical image data.
